Europäisches Patentamt

European Patent Office

Office européen des brevets

(19)

(11) Veröffentlichungsnummer: **0 148 303**
**B1**

(12)

# EUROPÄISCHE PATENTSCHRIFT

(45) Veröffentlichungstag der Patentschrift:
06.04.88

(21) Anmeldenummer: **84100207.4**

(22) Anmeldetag: **11.01.84**

(51) Int. Cl.⁴: **A 23 D 3/00,** A 23 L 1/30,
A 61 K 31/685

(54) **Diätetikum.**

(43) Veröffentlichungstag der Anmeldung:
**17.07.85 Patentblatt 85/29**

(45) Bekanntmachung des Hinweises auf die Patenterteilung:
**06.04.88 Patentblatt 88/14**

(84) Benannte Vertragsstaaten:
**AT BE CH DE FR GB IT LI LU NL SE**

(56) Entgegenhaltungen:
**GB-A-2 080 324**
**US-A-2 402 690**

(73) Patentinhaber: **von Mietzko, Armin, Dr.,
Waldwinkel 12, D-4520 Melle 1 (DE)**

(72) Erfinder: **Lingner, Lotte, Auf der Bischoffhöhe 64,
CH- 4125 Riehen (CH)**

(74) Vertreter: **Depmeyer, Lothar, Auf der Höchte 30,
D-3008 Garbsen 1 (DE)**

EP 0 148 303 B1

## Beschreibung

Die Erfindung betrifft ein Diätetikum mit Phospolipiden, insbes. Lecithin.

Das in Pflanzenölen vorkommende Lecithin kann eine biologisch günstige Zusammensetzung, dh. einen hohen Anteil ungesättigter Fettsäuren haben. Jedoch wird dieses Lecithin durch die heute üblichen Raffinationsverfahren vollständig entfernt. Falls sich noch Fetthärtung und Umesterung anschliessen, verliert auch das Fett selbst seinen Anteil an ungesättigten Fettsäuren. Als Folge für den Konsumenten dieses Fettes können sich vielfältige Störungen des Lipidhaushaltes und Stoffwechsels, Cholesterinablagerungen in den Blutgefässen, mangelhafte periphere Durchblutung, Arteriosklerose und Bluthochdruck einstellen. Diese Erscheinungen sind tatsächlich auf den Ernährungsmangel zurückzuführen.

Es ist ein Lecithin enthaltendes für diätetische Zwecke geeignetes Produkt bekannt (GB-A-2 080 324); dessen Fettgehalt beträgt minimal 5 % und maximal einer Konzentration entsprechend dem natürlichen Fettgehalt von plastischem Lecithin. Fett oder Fettsäuren werden nicht zugesetzt. Bei der Margarineherstellung ist es ferner bekannt (US-A-2 402 690), eine wasserhaltige Mischung zu benutzen, die Lecithin, weitere Emulgatoren und ein Fett enthält. Der Wassergehalt beträgt 5 - 10 %, der Emulgatorengehalt in der fertigen Margarine beträgt dabei bis zu 2 %. Eine direkte Nutzung dieses Produktes als Diätetikum ist nicht möglich. Die bekannten Produkte sind also entweder nicht als diätetische Mittel geeignet oder sie haben nicht die vergleichsweise hohe Konzentration an spezifisch mehrfach ungesättigten Säuren.

Ausgehend von den eingangs erwähnten Erkenntnissen liegt der Erfindung im wesentlichen die Aufgabe zugrunde, ein Diätetikum der eingangs genannten Art vorzuschlagen, mit dem bequem und angenehm in oraler Verabreichung in essentieller Form und in ausreichender Menge Phospholipide den inneren Systemen des menschlichen Organismus zugeführt werden können, um die Vorbeugung, die Linderung und die Beseitigung der erwähnten Störungen des menschlichen Lipidhaushaltes herbeiführen zu können.

Zur Lösung dieser Aufgabe enthält das Diätetikum erfindungsgemäss als homogene Mischung naben den Phospholipiden freie Fettsäuren und/oder fette Öle, die einen hohen Gehalt an Linolsäure, Linolensäure und Arachidonsäure aufweisen, wobei der Gehalt an Linolsäure, Linolensäure und Arachidonsäure bezogen auf den Gesamtfettsäuregehalt in Summe mindestans 50 % ausmacht und die Konzentration an Phospholipid in der Mischung etwa 5 - 45 % beträgt. Zweckmässigerweise beträgt hierbei die Konzentration an Phospholipiden in der Mischung jedoch 10 - 30 %. Ferner wird vorgeschlagen, dass die fetten Öle bzw. die Linolsäure, Linolensäure und Arachidonsäure im erfindungsgemässen Diätetikum einzeln oder im Gemisch vorgesehen sind.

Für das erfindungsgemässe Diätetikum wird weiter vorgeschlagen, dass es Baumwollsaatöl, Maiskeimöl, Sonnenblumenöl, Sojaöl, Distelöl, Walnussöl und Leinöl einzeln oder im Gemisch enthält. An Phospholipiden wird vorzugsweise Lecithin benutzt, jedoch können auch andere, basische Gruppen enthaltende Phospholipide wie Kephaline, Phosphatidylserin und Sphingomyeline zu Anwendung gelangen. Dabei soll jedoch aufgrund der Erfindung die Konzentration an Phospholipiden in der Mischung so gross sein, dass das Molverhältnis Phospholipid zu fettem Öl bzw. zu Linolsäure, Linolensäure und Arachidonsäure mindestens 1: 20 und höchstens 1: 1 beträgt. Dies bedeutet für eine aus Lecithin und Sonnenblumenöl bestehende Mischung eine Lecithinkonzentration von ca. 4 - 47 %.

Die Herstellung des Diätetikums erfolgt durch Mischen der Bestandteile bei normaler oder leicht erhöhter Temperatur (bis maximal 50° C) und nötigenfalls anschliessende Klärung durch Filtrieren oder Zentrifugieren. Die erhaltene homogene und klare Mischung stellt entweder als solche oder nach Vermengung mit weiteren, die Verabreichung begünstigenden Stoffen das Diätetikum dar. Die Weiterverarbeitung kann z. B. die Abfüllung in die Dosierung und die Einnahme erleichternde Einzeldosen (z. B. Gelantinekapseln) beinhalten oder aber auch in einer Weiterverarbeitung der Mischung zu Verdünnungen und Emulsionen (z. B. vom Typus Margarine) bestehen. Hierbei zugesetztes Fett muss wiederum einen Gehalt an mehrfach ungesättigten Fettsäuren von mind. 50 %, bezogen auf den Gesamtfettsäuregehalt, aufweisen.

Das Diätetikum kann in reiner oder verdünnter Form eingenommen werden. Bei direkter Verabreichung wird es zweckmässigerweise unmittelbar vor der Einnahme mit geeigneten Nahrungsmitteln vermischt. Seine besondere Zusammensetzung führt zu einem hohen Blutspiegel an "essentiellen Phospholipiden", d.h. solchen, mit mehrfach ungesättigten Fettsäuren im Molekül. Dies wird erreicht durch die gemeinsame Verabreichung mit mehrfach ungesättigten Fettsäuren bzw. mit fettem Öl, das reich an mehrfach ungesättigten Fettsäuren ist. Das Gemisch bildet mit wasserhaltigen Systemen, also z. B. normalen Speisen bzw. dem Mageninhalt, Emulsionen, die eine feine Verteilung und gute Verdauung und Resorption von Phospholipid und Öl bewirken. Wie bei allen Lipiden vom Glycerinester-typus Tindet hierbei zunächst eine Hydrolyse, dann die Resorption der Spaltstücke und schliesslich die Resynthese in der Darmmukosa statt. Bei der Resynthese vermag der Organismus bekanntlich Fettsäuren nicht zu selektionieren, sondern er bedient sich des gerade vorhandenen Angebots. Da die menschliche Nahrung überwiegend Fette mit gesättigten Fettsäuren enthält, besteht normalerweise das in der Darmmukosa zu einem beliebigen Zeitpunkt vorhandene Angebot ebenfalls überwiegend aus derartigen gesättigten Fettsäuren. Nach einer Verabreichung von z. B. unvermischtem Lecithin werden daher die Lecithin-hydrolyseprodukte nicht etwa zu dem ursprünglichen, linolsäurehaltigen Lecithin resynthetisiert, sondern es entsteht ein Lecithin mit Fettsäureresten im Verhältnis des vorhandenen Angebots, d. h. also eines mit hohem Gehalt an gesättigten Fettsäuren. Dieses ist aus folgendem Grund biologisch wertlos:

Lecithin vermag nur dann mit Wasser sich zu hydratisieren und in wässrigen Systemen sich micellar bzw. in Liposomenform zu lösen, wenn seine Alkylketten sich in nichtkristallinem, d.h. quasiflüssigen Zustand befinden. Dieser Zustand ist oberhalb einer für jedes Lecithin spezifischen Übergangstemperatur erreicht. Unterhalb dieser Temperatur liegen die Alkylketten in starrem, kristallinen Zustand vor, womit Quellung, Hydratisierung und Auflösung unterbunden werden. Lecithin mit Linolsäure als Fettsäurekomponente besitzt eine Übergangstemperatur von ca. 0°C, Lecithin mit gesättigten Fettsäuren demgegenüber eine solche von deutlich oberhalb der Körpertemperatur (ca. 70°C). Natürliches Lecithin mit gemischten Fettsäureresten (mindestens eine mehrfach ungesättigte Fettsäure pro Molekül) hat ebenfalls eine Übergangstemperatur, die noch unterhalb der Körpertemperatur liegt. Ausschliesslich diejenigen im Organismus resynthetisierten und an das Lymphsystem und Blutplasma abgegebenen Lecithine können als biologisch wertvoll oder "essentiell" bezeichnet werden, in denen, wie beim natürlichen Lecithin, mindestens einer der Fettsäureketten mehrfach ungesättigt ist. Dies wird bei der in unserer Gesellschaft üblichen Ernährungsweise durch die Verabreichung von Lecithin allein nicht bzw. in nur völlig unzureichendem Masse erreicht. Aus den erwähnten Gründen ist hierfür die gleichzeitige Zufuhr von mehrfach ungesättigten Fettsäuren bzw. von Fetten mit hohem Gehalt an mehrfach ungesättigten Fettsäuren nötig.

In die Blutbahn gelangende essentielle (mehrfach ungesättigte Fettsäuren enthaltende) Phosphatide vermögen einen gestörten Lipidhaushalt zu normalisieren. Speziell sind sie in der Lage, Cholesterinablagerungen in den Blutgefässen zu verhindern bzw. wieder aufzulösen. Arteriosklerotisch bedingter Bluthochdruck und Folgeerscheinungen mangelnder peripherer Durchblutung bilden sich zurück. Das an sich wasserunlösliche Cholesterin z. B. ist in einer Lecithin-Gallensäure-Wasser-Mischung bis zu einem Molverhältnis von 1:1 (Cholesterin zu Lecithin) löslich, was gewichtsmässig ungefähr 0,5:1 entspricht. In der Blutbahn enthaltenes essentielles Lecithin schafft somit Bedingungen, die die Löslichkeit und Transportierbarkeit von Cholesterin aufrechterhalten bzw. die Wiederauflösung entsprechender Ablagerungen ermöglichen. Schliesslich ist der quantitative Aspekt wesentlich. Der menschliche Organismus benötigt täglich ca. 2 - 3 g Cholesterin und muss bei einem gestörten Lipidhaushalt das Lösungsvermögen seines Blutplasmas für Cholesterin kräftig und über längere Zeit hinweg erhöhen. Die tägliche Zufuhr an essentiellem, in die Blutbahn gelangendem Lecithin sollte in einem solchen Fall daher 1 g nicht unterschreiten, besser aber ca. 5 g betragen. Diese Dosierung ist mit dem Diätetikum bequem erreichbar.

Zu empfehlen ist die Anwendung des Diätetikums als Speisezusatz. Es ist in diesem Fall geschmacksneutral bis angenehm schmeckend. Andere Fette sollten den entsprechenden Speisen nicht hinzugefügt werden bzw. nur solche, deren Gehalt an mehrfach ungesättigten Fettsäuren ebenfalls hoch ist. Dasselbe gilt sinngemäss für kurz vor und nach der Einnahme des Diätetikums zugeführte Nahrungsmittel.

Nach einem weiteren Vorschlag gemäss der Erfindung dient das Diätetikum als Ausgangsmateriel zur Herstellung von Diätmargarine, deren Konzentration an Phospholipiden - bezogen auf den Gesamtfettgehalt - etwa 5 - 45 %, vorzugsweise jedoch 10 - 40 % beträgt.

Das Diätetikum eignet sich auch sehr gut als Vehikel, um dem menschlichen Organismus fettlösliche Mangelstoffe anderer Art zuzuführen wie z. B. fettlösliche Vitamine und Provitamine.

Unter "Lecithin" wird in dieser Patentschrift das handelsübliche, natürliche Gemisch von Phospholipiden verstanden, das normalerweise aus rohem Sojaöl gewonnen wird und neben Lecithin in engerem Sinne ( = Phosphatidylcholin) weitere Phospholipide wie Kephaline (Phosphatidylcolamin und Phosphatidylserin) sowie Inositphosphatide enthält. Daneben können aber auch handelsübliche Lecithinverdünnungen, Phospholipidgemische aus anderen Quellen sowie mehr oder weniger angereicherte Lecithinpräparate verwendet werden. Alle in dieser Patentschrift für "Lecithin" gemachten Konzentrationsangaben beziehen sich auf den Gehalt an Gesamtphospholipiden.

Zu empfehlen sind als Ausgansprodukte für das erfindungsgemässe Diätetikum Lecithinverdünnungen enthaltend ca. 30 bis 70 % Gesamtphospholipide neben fettem Öl mit einem genügend hohen Gehalt an Linolsäure, Linolensäure und Arachidonsäure, wobei diese Verdünnungen direkt oder nach einer weiteren Verdünnung mit geeigneten fetten Ölen das Diätetikum darstellen unter der Bedingung, dass dessen Zusammensetzung dem Diätetikum gemäss Ansprüche 1 und/oder 4 entspricht.

Es ist ferner möglich dem Diätetikum fettlösliche Vitamine (A, D, E, K) oder die entsprechenden Provitamine zuzusetzen. Insb. wird vorgeschlagen, ein Diätetikum zu verabreichen, das pro Tagesdosis einzeln oder in Mischung 0.5- 2 mg Vitamin A palmitat, 2 bis 10 mg β-Carotin, 0.01 bis 0.1 mg Vitamin $D_2$ und/oder 50 bis 200 mg α-Tokopherolazetat enthält.

**Patentansprüche**

1. Diätetikum mit Phospholipiden, insb. Lecithin, dadurch gekennzeichnet, dass es als homogene Mischung neben den Phospholipiden freie Fettsäuren und/oder fette Öle die einen hohen Gehalt an Linolsäure, Linolensäure und Arachidonsäure aufweisen, enthält, wobei der Gehalt an Linolsäure, Linolensäure und Arachidonsäure bezogen auf den Gesamtfettsäuregehalt in Summe mindestens 50 % ausmacht und die Konzentration an Phospholipid in der Mischung etwa 5 - 45 % beträgt.

2. Diätetikum nach Anspruch 1, dadurch gekennzeichnet, dass die fetten Öle bzw. die Linolsäure, Linolensäure und Arachidonsäure einzeln oder im Gemisch vorgesehen sind.

3. Diätetikum nach Anspruch 1, dadurch gekennzeichnet, dass es Baumwollsaatöl, Maiskeimöl, Sonnenblumenöl, Sojaöl, Distelöl, Walnussöl und Leinöl einzeln oder im Gemisch enthält.

4. Diätetikum nach Anspruch 1, dadurch gekennzeichnet, dass die Konzentration an Phospholipiden in der Mischung so gross ist, dass das Molverhältnis Phospholipid zu fettem Öl bzw. zu Linolsäure, Linolensäure und Arachidonsäure mindestens 1: 20 und höchstens 1: 1 beträgt.

5. Diätetikum nach Anspruch 1, dadurch gekennzeichnet, dass die Konzentration an Phospholipiden in der Mischung 10 - 30 % beträgt.

6. Diätetikum nach Anspruch 1, dadurch gekennzeichnet, dass es als Ausgangsmaterial zur Herstellung von Diätmargarine dient, deren Konzentration an Phospholipiden-bezogen auf den Gesamtfettgehalt - etwa 5 - 45 % beträgt.

7. Diätetikum nach Anspruch 6, dadurch gekennzeichnet, dass die Konzentration an Phospholipiden - bezogen auf den Gesamtfettgehalt - 10 - 40 % beträgt.

8. Verfahren zur Herstellung eines Diätetikums nach Anspruch 1 und/oder 4, dadurch gekennzeichnet, dass als Ausgangsprodukt Lecithinverdünnungen, enthaltend ca. 30 bis 70 % Gesamtphospholipide neben fettem ÖL mit einem genügend hohen Gehalt an Linolsäure, Linolensäure und Arachidonsäure, dienen, wobei diese Verdünnungen direkt oder nach einer weiteren Verdünnung mit geeigneten fetten Ölen das Diätetikum darstellen.

9. Diätetikum nach Anspruch 1, dadurch gekennzeichnet, dass fettlösliche Vitamine (A, D, E, K) oder die entsprechenden Provitamine zugesetzt sind.

10. Diätetikum nach Anspruch 9, dadurch gekennzeichnet, dass es pro Tagesdosis einzeln oder in Mischung 0.5 - 2 mg Vitamin A palmitat, 2 bis 10 mg β -Carotin, 0.01 bis 0.1 mg Vitamin $D_2$ und/oder 50 bis 200 mg α-Tokopherolazetat enthält.

**Claims**

1. Dietetic composition containing phospholipids, especially lecithin, characterised in that, in addition to the phospholipids, it contains free fatty acids and/or fatty oils, which have a high content of linoleic acid, linolenic acid and arachidonic acid, as a homogeneous mixture, the content of linoleic acid, linolenic acid and arachidonic acid totalling at least 50 % with respect to the total fatty acid content, and the concentration of phospholipid in the mixture amounts to approximately 5 - 45 %.

2. Dietetic composition according to Claim 1, characterised in that the fatty oils, or respectively the linoleic acid, linolenic acid and arachidonic acid, are provided individually or in combination with one another.

3. Dietetic composition according to Claim 1, characterised in that it contains cottonseed oil, corn oil, sunflower oil, soya-bean oil, thistle oil, walnut oil and linseed oil individually or in combination with one another.

4. Dietetic composition according to Claim 1, characterised in that the concentration of phospholipids in the mixture is so great that the molar ratio of phospholipid to fatty oil, or respectively to linoleic acid, linolenic acid and arachidonic acid, is at least 1: 20 and at most 1: 1.

5. Dietetic composition according to Claim 1, characterised in that the concentration of phospholipids in the mixture is 10 - 30 %.

6. Dietetic composition according to Claim 1, characterised in that it serves as the starting material for producing dietetic margarine having a concentration of phospholipids of approximately 5 - 45 % with respect to the total fat content.

7. Dietetic composition according to Claim 6, characterised in that the concentration of phospholipids is 10 - 40 % with respect to the total fat content.

8. Method of producing a dietetic composition according to Claim 1 and/or 4, characterised in that lecithin dilutions, containing approx. 30 to 70 % of the total phospholipids, in addition to fatty oil having a sufficiently high content of linoleic acid, linolenic acid and arachidonic acid, serve as the starting product, these dilutions constituting the dietetic composition directly or after a further dilution with suitable fatty oils.

9. Dietetic composition according to Claim 1, characterised in that fatsoluble vitamins (A, D, E, K) or the corresponding provitamins are added.

10. Dietetic composition according to Claim 9, characterised in that it contains, per daily dose, 0.5 - 2 mg vitamin A palmitate, 2 to 10 mg β-carotene, 0.01 to 0.1 mg vitamin $D_2$, and/or 50 to

200 mg α-tocopherol acetate individually or in combination with one another.

**Revendications**

1. produit diététique avec phospholipides, en particulier la lécithine, caractérisé en ce qu'il contient sous forme de mélange homogène, outre les phospholipides, des acides gras libres et/ou des huiles grasses, qui présentent une teneur élevée en acide linoléique, acide linolénique et acide arachidonique, où la teneur en acide linoléique, acide linolénique et acide arachidonique par rapport à la teneur totale en acides gras constitue au total au moins 50 % et la concentration en phospholipide dans le mélange s'élève à environ 5-45 %.

2. Produit diététique selon la revendication 1, caractérisé en ce que les huiles grasses ou selon les cas l'acide linoléique, l'acide linolénique et l'acide arachidonique sont prévus isolément ou mélangés.

3. Produit diététique selon la revendication 1, caractérisé en ce qu'il contient de l'huile de graine de coton, de l'huile de germe de maïs, de l'huile de tournesol, de l'huile de soja, de l'huile de chardon, de l'huile de noix et de l'huile de lin isolément ou mélangés.

4. Produit diététique selon la revendication 1, caractérisé en ce que la concentration en phospholipides dans le mélange est si importante que le rapport molaire du phospholipide à l'huile grasse ou selon les cas à l'acide linoléique, l'acide linolénique et l'acide arachidonique s'élève au moins à 1 : 20 et au plus à 1 : 1.

5. Produit diététique selon la revendication 1, caractérisé en ce que la concentration en phospholipides dans le mélange s'élève à 10-30 %.

6. Produit diététique selon la revendication 1, caractérisé en ce qu'il sert de produit de départ pour la préparation de margarines diététiques dont la concentration en phospholipides - par rapport à la teneur en matière grasse totale - s'élève à environ 5-45 %.

7. Produit diététique selon la revendication 6, caractérisé en ce que la concentration en phospholipides - par rapport à la teneur en matière grasse totale-s' élève à 10-40 %.

8. Procédé de préparation d'un produit diététique selon la revendication 1 et/ou 4, caractérisé en ce qu'on utilise comme produit de départ des dilutions de lécithine contenant d'environ 30 à 70 % de phospholipides totaux outre une huile grasse avec une teneur suffisamment élevée en acide linoléique, acide linolénique et acide arachidonique, ces dilutions représentant directement ou après une dilution plus poussée avec des huiles grasses appropriées le produit diététique.

9. Produit diététique selon la revendication 1, caractérisé en ce qu'on ajoute des vitamines (A, D, E, K) solubles dans les matières grasses ou les provitamines correspondantes.

10. Produit diététique selon la revendication 9, caractérisé en ce qu'il contient par dose quotidienne isolément ou en mélange 0,5-2 mg de palmitate de vitamine A, 2 à 10 mg de β-carotène, 0,01 à 0,1 mg de vitamine $D_2$ et/ou 50 à 200 mg d' α-tocophérolacétate.